# FASCICULE DE BREVET EUROPEEN

(11) **EP 0 362 766 B1**
(45) Date de publication et mention de la délivrance du brevet: **29.12.1993**
(21) Numéro de dépôt: 89118232.1
(22) Date de dépôt: 02.10.1989
(51) Int. Cl.: A61B 3/00, A61F 9/00

(54) **Dispositif de commande d'un appareil de traitement ophtalmologique**
Verstellgerät für eine ophthalmologische Behandlungsvorrichtung
Control device for an ophthalmological treatment apparatus

(30) Priorité: 06.10.1988 CH 3744/88; 10.10.1988 FR 8813402
(43) Date de publication de la demande: 11.04.1990
(73) Titulaire: HAAG-STREIT AG, CH-3098 Köniz (CH)
(72) Inventeur: Lawniczak, Michel, CH-3612 Steffisburg (CH); Meerstetter, Robert, CH-3604 Thun (CH); Bätscher, Paul, CH-3110 Münsingen (CH)
(74) Mandataire: de Raemy, Jacques

(56) Documents cités:
- EP-A- 0 091 334
- DE-A- 3 601 022
- GB-A- 2 154 306
- US-A- 4 470 320

## Description

La présente invention est relative à un dispositif de commande d'un appareil de traitement ophtalmologique, ledit appareil comportant au moins un faisceau laser de puissance présentant un point focal de concentration dudit faisceau susceptible d'agir, sur l'ordre de tir d'un praticien, à un endroit déterminé de l'oeil d'un patient, endroit défini par des première, deuxième et troisième coordonnées dimensionnelles orthogonales, ledit dispositif de commande comprenant un boîtier sur lequel peut reposer une main d'un praticien, ledit boîtier étant pourvu d'un premier moyen pour fixer le point focal selon la première coordonnée, d'un second moyen pour fixer le point focal dans un plan selon les deuxième et troisième coordonnées et d'un troisième moyen pour déclencher le tir laser quand lesdites trois coordonnées sont atteintes, lesdits premier, deuxième et troisième moyens étant arrangés de telle manière qu'ils puissent être commandés chacun par les doigts de la même main.

Un appareil de traitement ophtalmologique dont il s'agit d'assurer la commande est décrit par exemple dans le document EP-B-0 030 210. L'appareil comporte essentiellement une première source de lumière qui émet un faisceau laser de puissance destiné au traitement proprement dit et une seconde source de lumière qui émet un faisceau de lumière cohérente visible de faible puissance arrangé pour envelopper le faisceau de puissance. Les faisceaux mentionnés passent alors à travers une lentille convergente qui les concentre en un point focal qui est le point de traitement, le faisceau de lumière visible étant présent pour permettre de localiser avec précision l'endroit où se trouve le foyer du faisceau de puissance. Le point de focalisation est observé par un praticien à travers un oculaire (ou un binoculaire) alors que la cavité oculaire est éclairée par une source auxiliaire de lumière. Lorsque l'endroit recherché pour le traitement est atteint, le praticien commande le tir laser.

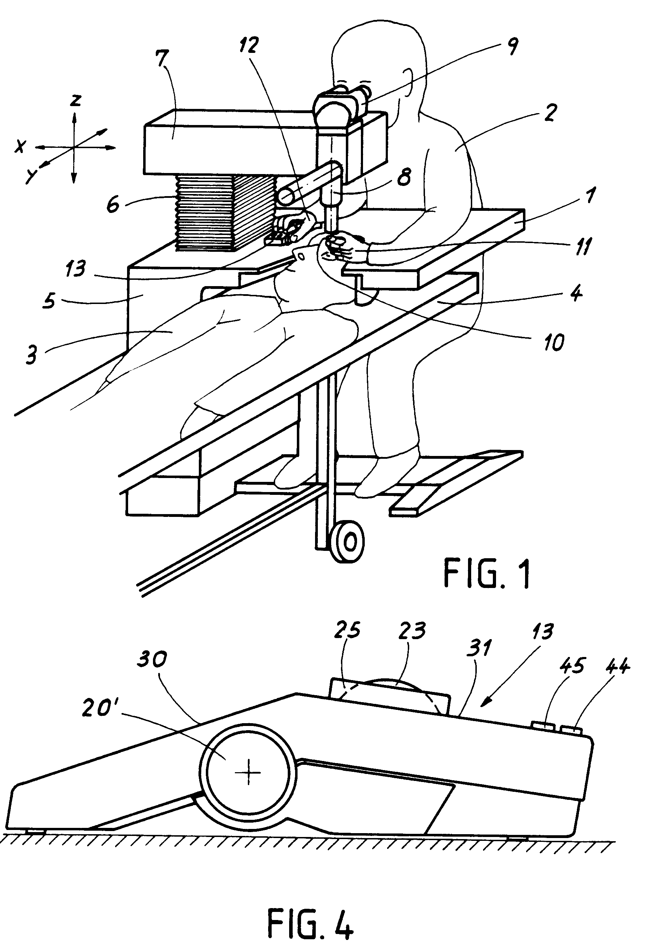

Les appareils de l'art antérieur se présentent dans les grandes lignes comme montré en figure 1. L'appareil de traitement comporte une table 1 devant laquelle est assis un praticien 2. De l'autre côté de la table prend place un patient 3, ici couché sur un lit 4 venant s'encastrer avec précision dans la table. Dans certains cas, le patient est assis et repose sa tête sur une mentonnière fixée à la table. Quelle que soit la position choisie pour le patient, l'appareil est conçu pour que la tête du patient soit immobilisée par rapport audit appareil.

La figure 1 montre encore un coffret 5 qui supporte la table. Ce support contient généralement les sources donnant naissance aux faisceaux de puissance et de lumière visible dont il a été question ci-dessus. Ces faisceaux sont acheminés à l'intérieur de l'oeil du patient par une colonne 6 et un bras mobile 7, ce dernier portant une optique de sortie 8 susceptible de focaliser les faisceaux en un point qu'il s'agit d'amener à un endroit bien précis de l'oeil du patient, point qui est observé par le praticien à travers le binoculaire 9. Dans ce but, le bras 7 peut se mouvoir selon les trois coordonnées orthogonales X, Y et Z.

Pour dévier les faisceaux sortant de l'optique 8 et les diriger à un point précis de la cavité oculaire, le praticien se sert d'un verre de contact 10 qu'il tient sur l'oeil du patient. Dans ce cas, sa main gauche 11 est occupée. La main droite 12 du praticien doit donc pouvoir à elle seule actionner un dispositif de commande 13, qui n'est pas décrit dans le document cité plus haut, et qui doit permettre la commande d'au moins quatre fonctions : celles du déplacement de la colonne 7 selon trois coordonnées X, Y et Z et celle du tir laser au moment où les trois coordonnées recherchées sont atteintes. On pourrait imaginer, sans que cela fasse preuve d'une activité inventive quelconque, que le dispositif de commande 13 comporte quatre organes distincts commandant respectivement les déplacements X, Y et Z et le tir laser, les trois premiers organes pouvant être des roulettes qu'on actionne dans les deux sens et le quatrième organe pouvant être un simple bouton-poussoir. Cette disposition est mal commode car elle oblige le praticien à mémoriser l'emplacement des roulettes et les fonctions qui leur sont dévolues avec les risques d'erreur ou de pertes de temps qu'un tel système peut entraîner, puisque la vue du praticien est entièrement occupée à observer la cavité de l'oeil à soigner et ne doit pas être distraite pour vérifier, par exemple, si le bon organe de commande est actionné.

Aussi, pour éviter les inconvénients énumérés ci-dessus, la présente invention est caractérisée soit par le fait que les premier et deuxième moyens sont une boule susceptible d'être respectivement enfoncée et roulée par un premier doigt et que le troisième moyen est un poussoir susceptible d'être enfoncé par un deuxième doigt, soit par le fait que le premier moyen est une roulette susceptible d'être tournée par un premier doigt, que le second moyen est une boule susceptible d'être roulée par un deuxième doigt et que le troisième moyen est un poussoir susceptible d'être enfoncé par un troisième doigt.

Un dispositif de commande associant une boule à un clavier d'ordinateur est montré dans le document GB-A-2 154 306. Dans ce document il n'est pas indiqué que la boule puisse être pressée ou associée à une roulette pour fixer le point focal selon une première coordonnée.

L'invention sera expliquée maintenant en se référant au dessin annexé qui l'illustre à titre d'exemple et dans lequel :
La figure 1 montre en perspective un appareil de traitement ophtalmologique qui utilise un dispositif de commande selon l'invention.
La figure 2 est une vue de dessus du dispositif de commande selon l'invention, desservi par la main droite d'un praticien droitier.
La figure 3 est une vue de dessus du dispositif de commande selon l'invention, desservi par la main gauche d'un praticien gaucher.
La figure 4 est une vue latérale du dispositif selon l'invention et selon la flèche IV de la figure 2.

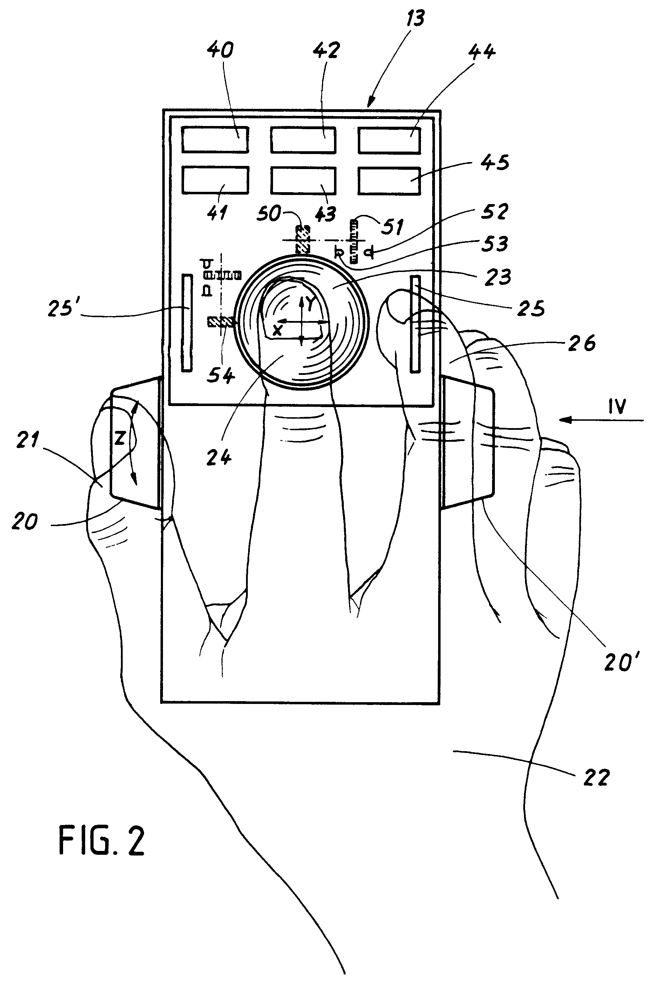

L'appareil de traitement ophtalmologique de la figure 1 a été décrit ci-dessus comme faisant partie de l'état de la technique. Cet appareil est commandé au moyen d'un dispositif 13 original qui va être décrit aux figures 2, 3 et 4.

Comme on le voit sur la figure 2, le dispositif 13 est un boîtier sur lequel a été représenté une main d'un praticien. Le dispositif est pourvu d'un premier moyen pour fixer le point focal du faisceau laser selon une première coordonnée. La figure montre que ce premier moyen est ici une roulette 20 qui peut être tournée par un premier doigt 21, en l'occurence le pouce de la main droite 22. La figure 2 montre encore que le dispositif est pourvu d'un second moyen pour fixer le point focal du faisceau laser dans un plan selon des deuxième et troisième coordonnées. Ce deuxième moyen est ici une boule 23 susceptible d'être roulée par un deuxième doigt 24, soit l'index de la main droite 22. Enfin la figure 2 montre que le dispositif comporte un troisième moyen pour déclencher le tir laser dès que lesdites trois coordonnées ont été atteintes par l'actionnement des premier et deuxième moyens. Ce troisième moyen se présente ici sous la forme d'un poussoir 25 susceptible d'être actionné par un troisième doigt 26, soit le majeur de la main droite 22. Tout à fait généralement, on s'aperçoit que les premier, deuxième et troisième moyens sont arrangés de telle manière qu'ils puissent être commandés chacun par un doigt différent de la même main.

On constate que le dispositif n'utilise que deux doigts pour amener le point focal du faisceau à l'endroit désiré et ceci grâce à l'utilisation d'un seul moyen (la boule) pour positionner le point focal dans un plan. L'utilisation de l'index pour assurer la recherche de deux coorodonnées dans un plan est particulièrement avantageuse à cause, d'une part, de l'excellente mobilité que présente ce doigt en comparaison de la mobilité des autres doigts de la main et, d'autre part, de la faculté d'indépendance d'action de ce doigt par rapport aux autres. L'utilisation du pouce pour la recherche d'une seule coordonnée est aussi judicieuse, ce doigt étant moins mobile, mais se prêtant bien à l'action de roulement qu'on lui demande d'effectuer sur la roulette. Enfin, l'utilisation du majeur pour le tir laser est avantageuse surtout du fait de sa position bien adaptée à actionner un bouton-poussoir.

Si l'on attribue à la première coordonnée, la fixation du point focal par rapport à la profondeur de l'oeil et aux deuxième et troisième coordonnées, la fixation du point focal dans un plan parallèle à la face du patient, on aura de plus une suite logique d'actions entreprises d'abord par le pouce, puis par l'index et enfin par le majeur lorsque les deux premiers doigts se seront fixés dans une position pour laquelle le tir peut avoir lieu, cette suite logique correspondant au rang logique qu'occupent le pouce, l'index et le majeur composant une main.

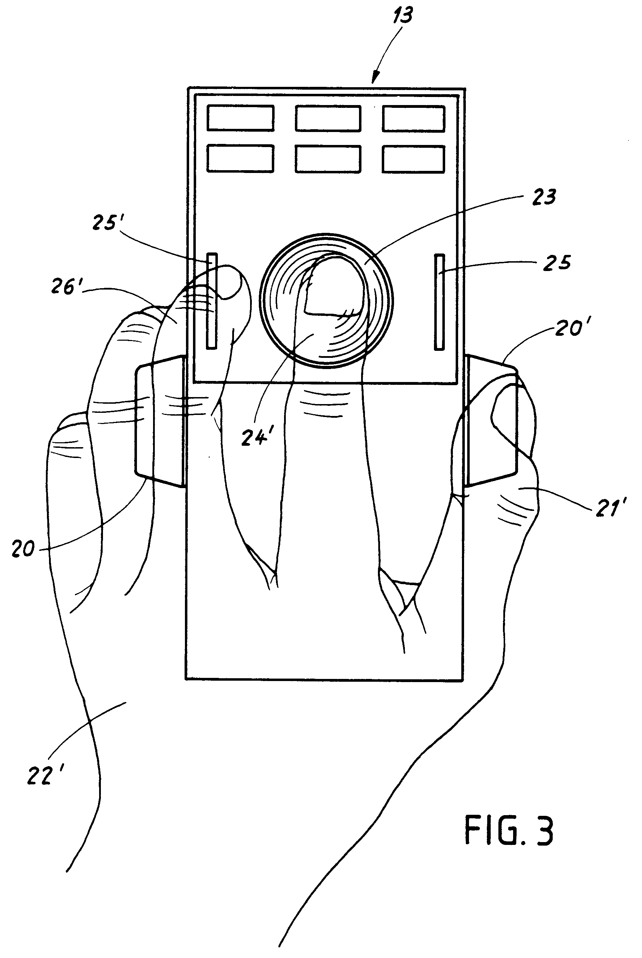

La figure 2 montre que la roulette 20 et le poussoir 25 sont dédoublés par une seconde roulette 20′ et un second poussoir 25′. Cette disposition permet d'utiliser le dispositif de commande également avec la main gauche comme cela est représenté en figure 3. En figure 3, la main gauche 22′ par son pouce 21′, son index 24′ et son majeur 26′ commande respectivement la roulette 20′, la boule 23 et le poussoir 25′. On retrouve en figure 3 la même disposition avantageuse des commandes, disposées également de façon toute logique mais pour la main gauche cette fois-ci.

Si l'on se réfère maintenant à la figure 4, qui est une vue selon la flèche IV de la figure 2, on retrouve sur le dispositif 13 les mêmes trois moyens de commande qui ont été décrits ci-dessus, c'est-à-dire dans l'ordre la roulette 20′, la boule 23 et le poussoir 25. Cette vue montre en outre une particularité supplémentaire de l'invention qui consiste à briser la face contre laquelle la main vient s'appuyer en une première zone montante 30 susceptible de servir d'appui au poignet de la main et en une seconde zone descendante 31 sur laquelle sont disposés la boule 23 et les poussoirs 25, 25′ commandés respectivement par l'index et le majeur de la main (non représentée ici). Cette dispositif en face brisée est particulièrement bien adaptée à l'appui de la main et contribue, si on y ajoute la disposition judicieuse des commandes proprement dites, à une ergonomie particulièrement bien choisie, facilitant une action rapide, dépourvue d'erreur et réduisant la fatigue tant physique qu'intellectuelle.

Les figures 2, 3 et 4 montrent que le dispositif de commande peut comporter en outre des quatrièmes moyens pour atteindre rapidement une zone se trouvant dans l'environnement proche de l'endroit où le tir doit avoir lieu. Il s'agit en fait de poussoirs permettant un préréglage grossier selon les trois coordonnées. Dans les figures, les poussoirs 40 et 41 permettent une recherche rapide de la première coordonnée. Il en va de même pour les poussoirs 42, 43 et 44, 45 qui permettent la même recherche rapide, mais pour les deuxième et troisième coordonnées, respectivement.

Le moyen pour déplacer dans un plan le point focal à l'aide d'un seul doigt consiste ici à utiliser une boule qui peut rouler librement autour de son centre. On pourrait envisager d'autres moyens par exemple l'utilisation d'un manche à balai, appelé communément "joy-stick" à la place de la boule. L'utilisation d'un joy-stick pose cependant des problèmes de stabilité de position et de précision.

La figure 2 montre qu'on a affecté à la boule les coordonnées X et Y. De façon généralement connue de l'état de la technique, la boule 23 entraîne deux roues 50, 54 disposées orthogonalement l'une par rapport à l'autre. La figure montre que la roue 50 entraîne un disque 51. Ce disque est percé de fentes qui laissent passer la lumière émise par une lampe 52. De l'autre côté du disque se trouve un capteur photoélectrique qui reçoit la lumière traversant les fentes. Les impulsions électriques issues du capteur commandent une électronique de puissance qui commande à son tour, par exemple, un moteur pas à pas. Ce moteur commande le bras 7 de l'appareil ophtalmologique montré en figure 1 et cela selon la coordonnée Y. Un dispositif semblable est mis en oeuvre pour le déplacement du bras selon la coordonnée X, à partir de la roue 54. La coordonnée Z est commandée par le pouce 21 agissant sur la roulette 20. L'angle de rotation de la roulette peut aussi être converti en impulsions électriques comme expliqué ci-dessus.

Le système de boule apte à déplacer un point dans un plan est connu aujourd'hui et est utilisé par exemple dans certains ordinateurs personnels (PC). Dans cette application, le système est inversé par rapport à celui décrit ici, c'est-à-dire que ce n'est pas un doigt qui déplace la boule, mais la main qui promène le boîtier sur une table, la boule se trouvant sous le boîtier et roulant quand on déplace le boîtier. Ce système est appelé communément "souris" et est décrit notamment dans la revue "Mini & Micros", No 305 / 22 juin 1988. On en trouve une réalisation sous la marque déposée "Logimouse" commercialisée par Logitech SA, CH-1143 Apples. Le système souris présente cependant l'inconvénient de demander beaucoup de place pour l'excursion du boîtier sur la table et de se salir rapidement. De plus, on conçoit qu'il est plus difficile d'immobiliser toute la main qu'un seul doigt de cette main quand la position de point focal est trouvée, surtout si un autre doigt doit encore commander une roulette (coordonnée Z). On ajoutera que le système proposé dans l'invention permet d'atteindre très rapidement le point recherché et ceci avec une précision élevée et un effort moins grand.

La boule 23 décrite ici est utilisée pour déplacer le point focal dans un plan XY. On conçoit cependant qu'elle pourrait également servir à déplacer ce point selon la coordonnée Z. Cette fonction supplémentaire peut facilement être remplie, si l'on adjoint à la boule une possibilité d'enfoncement qui peut agir sur un contact, lui-même mettant en marche un moteur d'entraînement du bras selon la coordonnée Z. Dans ces conditions, un seul doigt de la main suffit à commander le bras dans les trois directions, l'index 24, 24′ par exemple. Dans ces conditions également, la fonction de tir peut être remplie soit par le pouce 21, 21′, soit par le majeur 26, 26′.

La description qui vient d'être faite se base sur un appareil de traitement où le patient est couché. Le déplacement commandé par la boule est réalisé alors dans un plan perpendiculaire à la verticale. Si le patient est assis, on comprendra que le déplacement commandé par la boule devra se faire dans un plan parallèle à la verticale. Pour cela le dispositif de commande sera équipé, si nécessaire, d'un système de permutation des coordonnées.

## Revendications

1. Dispositif de commande (13) d'un appareil de traitement ophtalmologique, ledit appareil comportant au moins un faisceau laser de puissance présentant un point focal de concentration dudit faisceau susceptible d'agir, sur l'ordre de tir d'un praticien (2), à un endroit déterminé de l'oeil d'un patient (3), endroit défini par des première, deuxième et troisième coordonnées dimensionnelles orthogonales, ledit dispositif de commande comprenant un boîtier sur lequel peut reposer une main (22, 22') d'un praticien, ledit boîtier étant pourvu d'un premier moyen pour fixer le point focal selon la première coordonnée, d'un second moyen pour fixer le point focal dans un plan selon les deuxième et troisième coordonnées et d'un troisième moyen pour déclencher le tir laser quand lesdites trois coordonnées sont atteintes, lesdits moyens étant arrangés de telle manière qu'ils puissent être commandés chacun par les doigts de la même main, caractérisé par le fait que les premier et deuxième moyens sont une boule (23) susceptible d'être respectivement enfoncée et roulée par un premier doigt et que le troisième moyen est un poussoir (25, 25') susceptible d'être enfoncé par un deuxième doigt.

2. Dispositif de commande selon la revendication 1, caractérisé par le fait que les premier, deuxième et troisième moyens sont agencés de telle manière qu'ils puissent être commandés respectivement par l'index (24, 24') et le pouce (21, 21') ou le majeur (26, 26') de la main du praticien.

3. Dispositif de commande (13) d'un appareil de traitement ophtalmologique, ledit appareil comportant au moins un faisceau laser de puissance présentant un point focal de concentration dudit faisceau susceptible d'agir, sur l'ordre de tir d'un praticien (2), à un endroit déterminé de l'oeil d'un patient (3), endroit défini par des première, deuxième et troisième coordonnées dimensionnelles orthogonales, ledit dispositif de commande comprenant un boîtier sur lequel peut reposer une main (22, 22') d'un praticien, ledit boîtier étant pourvu d'un premier moyen pour fixer le point focal selon la première coordonnée, d'un second moyen pour fixer le point focal dans un plan selon les deuxième et troisième coordonnées et d'un troisième moyen pour déclencher le tir laser quand lesdites trois coordonnées sont atteintes, lesdits moyens étant arrangés de telle manière qu'ils puissent être commandés chacun par les doigts de la même main, caractérisé par le fait que le premier moyen est une roulette (20, 20') susceptible d'être tournée par un premier doigt, que le second moyen est une boule (23) susceptible d'être roulée par un deuxième doigt et que le troisième moyen est un poussoir (25, 25') susceptible d'être enfoncé par un troisième doigt.

4. Dispositif de commande selon la revendication 3, caractérisé par le fait que lesdits premier, deuxième et troisième moyens sont agencés de telle manière qu'ils puissent être commandés respectivement par le pouce (21, 21'), l'index (24, 24') et le majeur (26, 26') de la main du praticien.

5. Dispositif de commande selon la revendication 1 ou la revendication 3, caractérisé par le fait qu'il comporte en outre des quatrièmes moyens (40 à 45) pour atteindre rapidement une zone se trouvant dans l'environnement proche de l'endroit où le tir doit avoir lieu.

6. Dispositif de commande selon la revendication 1 ou la revendication 3, caractérisé par le fait que lesdits premier, deuxième et troisième moyens sont arrangés pour qu'ils puissent être commandés indifféremment par la main droite (22) ou par la main gauche (22') du praticien.

7. Dispositif de commande selon la revendication 4, caractérisé par le fait que le boîtier présente une forme parallélépipédique présentant, sur la face contre laquelle la main peut s'appuyer, une boule (23) pouvant être roulée par l'index de la main pour fixer les deuxième et troisième coordonnées et deux poussoirs (25, 25') situés de part et d'autre de la boule pour commander le tir laser par le majeur de la main, ledit boîtier présentant sur chaque côté de ses faces latérales une roulette (20, 20') pouvant être tournée par le pouce de la main pour fixer la première coordonnée.

8. Dispositif de commande selon la revendication 7, caractérisé par le fait que ladite face est brisée et présente une première zone montante (30) susceptible de servir d'appui au poignet de la main et une seconde zone descendante (31) sur laquelle sont disposés la boule et les deux poussoirs.

## Claims

1. Control arrangement (13) for an apparatus for ophtalmological treatment, said apparatus including at least one laser power beam having a focal point of concentration of said beam capable of acting when fired by a practitioner (2) on a predetermined location within the eye of a patient (3), said location being defined by first, second and third dimensional orthogonal coordinates, said control arrangement comprising a casing on which a hand (22, 22') of a practitioner may rest, said casing being provided with a first means for fixing the focal point according to the first coordinate, a second means for fixing the focal point within a plane according to the second and third coordinates and a third means for releasing the laser beam when said three coordinates are reached, said means being arranged in a manner such they may each be controlled by the fingers of the same hand, characterized by the fact that the first and second means comprise a ball (23) adapted to be respectively pressed in and rolled by a first finger and that the third means is a push button (25, 25') adapted to be pressed in by a second finger.

2. Control arrangement according to claim 1, characterized by the fact that the first, second and third means are arranged in a manner such that they may be controlled respectively by the index finger (24, 24') and the thumb (21, 21') or the middle finger (26, 26') of the hand of the practitioner.

3. Control arrangement (13) for an apparatus for ophtalmological treatment, said apparatus including at least one laser power beam having a focal point of concentration of said beam capable of acting when fired by a practitioner (2) on a predetermined location within the eye of a patient (3), said location being defined by first, second and third dimensional orthogonal coordinates, said control arrangement comprising a casing on which a hand (22, 22') of a practitioner may rest, said casing being provided with a first means for fixing the focal point according to the first coordinate, a second means for fixing the focal point within a plane according to the second and third coordinates and a third means for releasing the laser beam when said three coordinates are reached, said means being arranged in a manner such they may each be controlled by the fingers of the same hand, characterized by the fact that the first means is a roller (20, 20') adapted to be rotated by a first finger, that the second means is a ball (23) adapted to be rolled by a second finger and that the third means is a push button (25, 25') adapted to be pressed in by a third finger.

4. Control arrangement according to claim 3, characterized by the fact that the first, second and third means are arranged in a manner such that they may be controlled respectively by the thumb (21, 21'), the index finger (24, 24') and the middle finger (26, 26') of the hand of the practitioner.

5. Control arrangement according to claim 1 or claim 3, characterized by the fact that it further comprises fourth means (40 to 45) for rapidly reaching a zone within the surroundings close to the location where release of the laser beam is to take place.

6. Control arrangement according to claim 1 or claim 3, characterized by the fact that said first, second and third means are arranged in a manner such that they may be indifferently controlled by the right hand (22) or the left hand (22') of the practitioner.

7. Control arrangement according to claim 4, characterized by the fact that the casing has the form of a parallelepiped which on the face on which the hand may rest exhibits a ball (23) which may be rolled by the index finger of the hand in order to fix the second and third coordinates and two push buttons (25, 25') located on either side of the ball so as to control firing of the laser beam by the middle finger of the hand, said casing exhibiting on each of its lateral faces a roller (20, 20') adapted to be rotated by the thumb of the hand in order to fix the first coordinate.

8. Control arrangement according to claim 7, characterized by the fact that said face is broken and exhibits a first rising zone (30) adapted to serve as a wrist support for the hand and a second falling zone (31) on which are arranged the ball and the two push buttons.

## Patentansprüche

1. Steuervorrichtung (13) eines ophthalmologischen Behandlungsgerätes, das mindestens einen Leistungslaserstrahl umfaßt mit einem Konzentrationsbrennpunkt, mittels dem bei Auslösung durch einen Praktiker (2) an einer bestimmten Stelle des Auges eines Patienten (3) eingewirkt werden kann, welche Stelle definiert ist durch erste, zweite und dritte orthogonale räumliche Koordinaten, welche Steuervorrichtung ein Gehäuse umfaßt, auf dem eine Hand (22, 22') eines Praktikers ruhen kann, welches Gehäuse mit einem ersten Mittel versehen ist zum Festlegen des Brennpunktes gemäß der ersten Koordinate, mit einem zweiten Mittel zum Festlegen des Brennpunktes in einer Ebene gemäß der zweiten und dritten Koordinate und einem dritten Mittel zum Auslösen des Laserschusses, wenn die drei Koordinaten erreicht sind, welche Mittel derart angeordnet sind, daß jedes von ihnen von den Fingern derselben Hand betätigbar ist, dadurch gekennzeichnet, daß das erste und zweite Mittel eine Kugel (23) sind, die eindrückbar bzw. rollbar ist mittels eines ersten Fingers und daß das dritte Mittel ein Taster (25, 25') ist, der von einem zweiten Finger eindrückbar ist.

2. Steuervorrichtung nach Anspruch 1, dadurch gekennzeichnet, daß die ersten, zweiten und dritten Mittel derart ausgebildet sind, daß sie von dem Zeigefinger (24, 24') bzw. dem Daumen (21, 21') oder dem Mittelfinger (26, 26') der Hand des Praktikers betätigbar sind.

3. Steuervorrichtung (13) eines ophthalmologischen Behandlungsgerätes, das mindestens einen Leistungslaserstrahl mit einem Konzentrationsbrennpunkt aufweist, der bei Auslösung durch einen Praktiker (2) an einer bestimmten Stelle des Auges eines Patienten (3) einwirkt, welche Stelle definiert ist durch erste, zweite und dritte orthogonale räumliche Koordinaten, welche Steuervorrichtung ein Gehäuse umfaßt, auf dem eine Hand (22, 22') eines Praktikers ruhen kann, welches Gehäuse mit einem ersten Mittel versehen ist zum Festlegen des Brennpunktes gemäß der ersten Koordinate, mit einem zweiten Mittel zum Festlegen des Brennpunktes in einer Ebene entsprechend der zweiten und dritten Koordinate und einem dritten Mittel zum Auslösen des Laserschusses, wenn die drei Koordinaten erreicht sind, welche Mittel derart ausgebildet sind, daß jedes von ihnen von den Fingern derselben Hand betätigbar ist, dadurch gekennzeichnet, daß das erste Mittel eine Rolle (20, 20') ist, die von einem ersten Finger drehbar ist, daß das zweite Mittel eine Kugel (23) ist, die von einem zweiten Finger rollbar ist und daß das dritte Mittel ein Taster (25, 25') ist, der von einem dritten Finger eindrückbar ist.

4. Steuervorrichtung nach Anspruch 3, dadurch gekennzeichnet, daß die ersten, zweiten und dritten Mittel derart ausgebildet sind, daß sie von dem Daumen (21, 21'), dem Zeigefinger (24, 24') bzw. dem Mittelfinger (26, 26') der Hand des Praktikers betätigbar sind.

5. Steuervorrichtung nach Anspruch 1 oder Anspruch 3, dadurch gekennzeichnet, daß sie ferner vierte Mittel (40 bis 45) umfaßt zum schnellen Erreichen einer Zone, die sich in der nahen Umgebung der Stelle befindet, wo der Schuß stattfinden soll.

6. Steuervorrichtung nach Anspruch 1 oder Anspruch 3, dadurch gekennzeichnet, daß die ersten, zweiten und dritten Mittel so angeordnet sind, daß sie sowohl von der rechten Hand (22) wie auch der linken Hand (22') des Praktikers betätigbar sind.

7. Steuervorrichtung nach Anspruch 4, dadurch gekennzeichnet, daß das Gehäuse eine parallelepipedische Form aufweist, die auf der Seite, auf der die Hand sich abstützen kann, eine Kugel (23) aufweist, die vom Zeigefinger der Hand rollbar ist zum Festlegen der zweiten und dritten Koordinaten, sowie zwei Taster (25, 25') aufweist, die beidseits der Kugel angeordnet sind zum Steuern des Laserschusses durch den Mittelfinger der Hand, welches Gehäuse auf jeder Seite ihrer Seitenflächen eine Rolle (20, 20') aufweist, die von dem Daumen der Hand betätigbar ist, um die erste Koordinate festzulegen.

8. Steuervorrichtung nach Anspruch 7, dadurch gekennzeichnet, daß die genannte Seite abgeknickt ist und eine erste ansteigende Zone (30) aufweist, die als Auflage für das Gelenk der Hand dienen kann, und eine zweite absteigende Zone (31) aufweist, auf der die Kugel und die beiden Taster angeordnet sind.
